# EUROPEAN PATENT SPECIFICATION

(11) **EP 2 594 248 B1**
(45) Date of publication and mention of the grant of the patent: **14.12.2016**
(21) Application number: 13151581.9
(22) Date of filing: 18.02.2009
(51) Int. Cl.: A61K 8/34, A61K 8/49, A61K 8/67, A61Q 17/00, A61Q 15/00, A61Q 5/00, A61Q 19/00

(54) **Active ingredient combination and use thereof**
Wirkstoffkombination und ihre Verwendung
Constituants actifs combinés et leur utilisation

(30) Priority: 28.02.2008 DE 102008011692
(43) Date of publication of application: 22.05.2013
(62) Divisional of application: 09714386.1
(73) Proprietor: Air Liquide Santé (International), 75007 Paris (FR); SCHÜLKE & MAYR GMBH, 22851 Norderstedt (DE)
(72) Inventor: Beilfuss, Wolfgang, 22339 Hamburg (DE); Weber, Klaus, 20146 Hamburg (DE); Gradtke, Ralf, 25436 Tornesch (DE); Herweg, Sabine, 22844 Norderstedt (DE)
(74) Representative: Conan, Philippe Claude

(56) References cited:
- EP-A- 1 468 700
- DE-A1- 10 028 638
- DE-C1- 4 240 674
- FR-A- 2 729 050

## Description

The present invention relates to an active ingredient combination and to its use for producing a preparation in particular for the treatment of undesired body odour. Furthermore, the invention relates to preparations with a content of the combination.

Body odour is the term used to refer to smellable human body emanations from the skin and in the wider sense also from the body openings such as e.g. mouth odour. The most clearly perceptible is the smell of perspiration where only the secretions from the apocrine sweat glands smell, which are located primarily in the armpits. Fresh perspiration is completely odourless. Only the degradation of long-chain fatty acids to shorter chains such as formic acid or butyric acid causes the typical perspiration odour. A variety of bacteria are involved in this; these occur naturally on the skin.

To treat undesired body odour, in particular undesired body odour of perspiration, the following fundamental methods can be followed:
1. The undesired odour can be concealed through the use of odorants (fragrances, perfumes).
2. Odour absorbers are able to absorb odorants.
3. Antimicrobial agents can be used to control bacteria responsible for the decomposition of perspiration.
4. Perspiration inhibitors (antiperspirants) are substances which directly influence the secretion of perspiration and are thus also intended to prevent perspiration odour.

It is known to use 5-chloro-2-(2,4-dichlorophenoxy)-phenol (international non proprietary name: triclosan) as a bacteria inhibitor. However, the German Federal Institute for Risk Assessment advises against the use of triclosan in the home since bacterial resistance is suspected. Furthermore, polychlorinated dibenzodioxins and dibenzofurans can form from triclosan under solar irradiation.

It is known from DE 42 40 674 C1 that glycerol monoalkyl ethers of the formula ROCH₂CHOHCH₂OH have a good deodorizing effect. Preference is given to using 1-(2-ethylhexyl) glycerol ether (commercial product Sensiva^{®} SC 50, Schülke & Mayr GmbH). The patent specification discloses, inter alia, a deodorant formulation which comprises 58% by weight of water, 40% by weight of ethanol and 1.0% by weight of 1,2-propylene glycol, 0.9% by weight of 2-ethylhexyl glycerol ether and 0.1% by weight of bispyridinium alkane (octenidine dihydrochloride). This formulation is not sufficiently storage-stable. The result is the formation of peroxides and consequently the formation of degradation products, such as volatile substances with a low molecular weight and unpleasant smelling alcohols, such as 2-ethylhexanol.

Moreover, the content of volatile substances (VOC, such as ethanol) and the active ingredient content in the finished preparation are comparatively high.

Moreover, DE 10 2005 002 644 A1 discloses preparations for application in hygienic hand disinfection and disinfecting hand washing. The preparations comprise glycerol monoalkyl ether, bispyridinium alkane and polyol.

Accordingly, it was an object of the present invention to provide an active ingredient combination for preparations which are effective against those microorganisms which adversely affect, even in mild cases, the wellbeing of the user (e.g. unpleasant body odour, blemished, oily skin, comedones, dandruff, foot odour). The combination should also be storage-stable at a comparatively high concentration, even at elevated temperature, without formation of decomposition products or reduction in active ingredient content. The preparations furnished with the active ingredient combination should have the lowest possible VOC content and be sufficiently effective at a low active ingredient content and stable for as long as possible without decomposing.

Surprisingly, it has now been found that this object is achieved by an active ingredient combination according to Claim 1. The individual active ingredients a) glycerol ether and b) bispyridinium alkane are in each case in aqueous preparation soluble or clearly soluble and/or soluble or storage-stable at low temperatures only to a limited degree. By contrast, combinations according to the invention are (even at low temperatures) significantly more (storage)-stable and/or comprise active ingredient concentrations which are significantly above the solubility of the individual active ingredients (mutual solubility improvement of the components, improved concentrate stability).

According to the invention, combinations (typical combinations are concentrates) and preparations (typically diluted, e.g. aqueous, ready-to-use solutions) which are free from aromatic alcohols such as 2-phenoxyethanol, benzyl alcohol, phenethyl alcohol, 1-phenoxypropan-2-ol, 3-(4-chlorophenoxy)-1,2-propanediol, chlorobutanol, 2,4-dichlorobenzyl alcohol or mixtures thereof are preferred. Moreover, preference is given to combinations and preparations which comprise no salt selected from the salts of benzoic acid, propionic acid, salicylic acid, sorbic acid, 4-hydroxybenzoic acid, dehydracetic acid and 10-undecylenic acid.

Furthermore, in the case of combinations and preparations according to the invention, preference is given to an embodiment in which no aliphatic alcohol (no aliphatic, monohydric alcohol) and no amine oxide is present, as is obligatory according to DE 10 2005 002 645 A1 and DE 10 2005 002 643 A1. A content of aliphatic alcohol increases the amount of volatile substances and thus contributes to a high VOC content, which is undesired. In the case of the presence of amine oxides, there is the potential risk of the formation of nitrosamines, which is likewise undesired.

The content of component a) is generally in the range from 14 to 18% by weight, for example about 16% by weight, of component a), based on the active ingredient combination.

In a preferred embodiment, the content of component b) is in the range from 1.4 to 1.8% by weight, such as, for example, about 1.6% by weight, of component b), based on the active ingredient combination.

The improvement in the solubility of glycerol ethers in aqueous systems in the presence of bispyridinium alkanes is of importance in particular for relatively long-chain, very sparingly water-soluble glycerol ethers (e.g. for batyl alcohol, chimyl alcohol, selachyl alcohol, dodecyl glycerol ether, mentyl glycerol ether, since as a result of this their use in cosmetic (water-based) preparations is made considerably easier, e.g. without the assistance of relatively large amounts of solubility promoters.

According to the invention, as a component c) which can be used, 1,2-propylene glycol is particularly preferred.

The polyols act as (liquid) carriers, low-temperature stabilizers, solvents, solubility promoters, crystallization inhibitors, humectants (where the effect as skin humectant is additionally enhanced by the presence of the glycerol ethers). Here, the polyols, glycerol ethers and/or bispyridinium alkanes are able to mutually enhance their effects (e.g. deodorant efficacy, efficacy against problem germs, skin care effect).

In a preferred embodiment, the content of component c) is in the range from 70 to 85% by weight, 80 to 84% by weight, for example about 82 to 83% by weight, of component c), based on the active ingredient combination.

As component d), vitamin E and 3-tert-butyl-4-hydroxyanisole are preferred, and vitamin E and its derivatives are particularly preferred.

Suitable vitamin E forms (tocopherols) are, for example, naturally occurring vitamin E, synthetic vitamin E, enantiomerically pure forms of vitamin E (e.g. (+)-alpha-tocopherol), vitamin E derivatives such as acetates, succinates, linoleate, more water-soluble forms of vitamin E such as tocophereth-5, tocophereth-10, tocophereth-12, tocophereth-18, tocophereth-50, D-alpha-tocopherol polyethylene glycol 1000-succinates. Particular preference is given to synthetic vitamin E and naturally occurring vitamin E.

Here, a particularly preferred amount of antioxidant is 0.004 to 0.015% by weight or 0.005 to 0.01% by weight, such as about 0.008% by weight, based on the active ingredient combination according to the invention.

According to the invention, particular preference is given to a combination according to Claim 4. Combinations according to the invention can be water-containing, with preferred combinations comprising less than 55% by weight of water, such as less than 50% by weight, less than 40% by weight, less than 30% by weight, less than 20% by weight, less than 10% by weight, less than 5% by weight, where a particularly preferred combination comprises less than 5% by weight of water, such as less than 1% by weight of water, for example less than 0.5% by weight of water, where a particularly preferred combination according to the invention is free from water.

The combination according to the invention consists of components a) to d).

In a preferred embodiment of the combination according to the invention, the weight ratio of the sum of components a), b) and d) to component c) is 1:3 to 1:6. In a particularly preferred embodiment, the weight ratio is about 1:4 to 1:5.

In the case of preferred combinations, the weight ratio of component d) to component a) is 10:1 to 1:100 000, 1:1 to 1:50 000, 1:10 to 1:20 000, 1:100 to 1:10 000, 1:500 to 1:6000, 1:1000 to 1:4000, where in the case of particularly preferred combinations the weight ratio of component d) to component a) is about 1:2000.

The weight ratio of component a) to component b) is preferably 1:10 to 100:1, such as 1:1 to 50:1, 2:1 to 50:1 or 5:1 to 20:1, with particular preference being given to a combination in which the weight ratio of component a) to component b) is about 10:1.

The combinations according to the invention are typically clear, colourless solutions with a weak intrinsic odour. The solutions are storage-stable.

In a further aspect of the invention, the described combinations are used as concentrates for producing preparations. This use enables the preparations to be equipped with good efficacy against odour-causing germs. The following effects are achieved and/or the following odour-causing germs are effectively controlled according to the invention:
1. Mouth odour causing germs. Mouth odour is formed by volatile sulphur compounds, e.g. through bacterial decomposition of food remains or dead tissue, is e.g. caused by gram-negative anaerobes such as *Fusobacterium* species.
2. Perspiration odour causing germs. This is caused e.g. by the following skin germs, which are effective as odour germs: *Staphylococci spp.* (e.g. *epidermidis, aureus, hominis), Corynebacteria spp.* (e.g. *xerosis,* CDC G2), *Micrococcus spp.* (e.g. *Micrococcus luteus* = *Kokuria rhizophila*), *Anaerococcus spp.* (e.g. *octavius*). According to the invention, germs are preferably controlled which are responsible especially for the development of perspiration odour, for example *S. aureus, S. epidermis, M. luteus, P. mirabilis, K. pneumoniae* and *E. gergoviae.*
3. Body odour causing germs. Body odour is caused in particular by *Staph. hominis, Anaerococcus octavius* and *Corynebacterium spp.* (e.g. *xerosis,* CDC G2).
4. Foot odour causing germs. Foot odour is caused, for example, by microorganisms (bacteria, yeasts, fungi) such as *Brevibacteria spp., Trichophyton spp.,* e.g. *T. rubrum, T. mentagrophytes, Epidermophyton floccosum* etc.

The preparations are preferably used for the treatment (control, suppression) of undesired body odour. Typically, the preparations are cosmetic deodorant preparations.

Besides the excellent efficacy of the preparations furnished with the combination against deodorant germs, the good efficacy against problem germs such as propionibacterium acnes (suitable for preparations for the treatment of blemished skin, acne), Malassezia furfur (suitable for preparations to combat dandruff) and Trichophyton rubrum and Trichophyton mentagrophytes (suitable for preparations to combat foot and skin fungi) should also be emphasized. Foot fungus is one of the best known dermatomycoses and is a contagious fungal infection caused by dermatophytes and occurs in areas where heat and moisture accumulate. A foot fungus disorder manifests itself in itching, slightly reddened and flaking skin areas and also weeping blister formation. Accordingly, the invention also relates to the use of the combination for producing a (cosmetic or pharmaceutical) preparation for the treatment of blemished skin, acne, dandruff and/or foot and skin fungus.

The preparations comprise typically 0.01 to 5% by weight, preferably 0.05 to 1% by weight and for example about 0.1% by weight, of the combination present as concentrate as well as optionally one or more active ingredients, auxiliaries and/or additives.

Possible optional active ingredient(s), auxiliary(ies) and/or additive(s) are preferably: antidandruff active ingredients such as Octopirox™, Climbazol™ etc., deodorant active ingredients such as phenoxyethanol, polyhexamethylenebiguanide salts, chlorhexidine salts, triclosan, preservative active ingredients, bactericides, acylamino acids such as Lipacide C8G, fragrances, perfume oils, dyes, pigments, thickeners, surface-active substances, anionic, amphoteric, cationic or nonionic surfactants, emulsifiers, emollients, humectant substances, photoprotective agents (UVA filters, UVB filters, inorganic pigments such as ZnO), lipids, oils, essential oils (e.g. tee tree oil), plant extracts, polymers such as cellulose derivatives, polyacrylates, cationic polymers such as chitosan or chitosan derivatives, electrolytes, vitamins, biotin, penthenol, hair growth agents, solvents such as (preferably) water, alcohols, glycol ethers, pH regulators, buffers, foam stabilizers, carboxylic acids and salts thereof, hydroxy carboxylic acids and salts thereof, complexing agents, natural substances, thickeners, amino acids, proteins, protein hydrolyzates, carbohydrates and carbohydrate derivatives, polysaccharides, care substances or other customary constituents of a cosmetic formulation.

Examples of surface-active substances which can be used advantageously according to the invention are conventional soaps, e.g. fatty acid salts of sodium, alkyl sulphates, alkyl ether sulphates, alkane- and alkylbenzenesulfonates, sulfoacetates, sulfobetaines, sarcosinates, amidosulfobetaines, sulfosuccinates, sulfosuccinic acid half-esters, alkyl ether carboxylates, protein-fatty acid condensates, alkylbetaines and amidobetaines, fatty acid alkanolamides, polyglycol ether derivatives.

Preference is given to nonionic, amphoteric or cationic surface-active substances whose concentration can be between 0.1 and 40% by weight, preferably between 0.5 and 20% by weight, more preferably between 1 and 10% by weight, based on the preparation.

According to the invention, particular preference is given to a preparation which comprises
a) 0.008 to 0.03% by weight, e.g. 0.014 to 0.018% by weight, such as about 0.016% by weight, of glycerol monoalkyl ether (such as 1-(2-ethylhexyl) glycerol ether),
b) 0.0008 to 0.003% by weight, e.g. 0.0014 to 0.0018% by weight, such as about 0.0016% by weight, of bispyridinium alkane (such as octenidine dihydrochloride),
c) 0.04 to 0.15% by weight, e.g. 0.07 to 0.085% by weight, such as about 0.0825% by weight, of polyol (such as 1,2-propylene glycol) and
d) 30 to 200 ppb, e.g. 60 to 100 ppb, such as about 80 ppb, of antioxidant (such as vitamin E).

In one preferred embodiment of the invention, the preparations are formulated for topical application in the form of emulsions (e.g. creams, lotions), gels, solutions, embrocations, ointments, sprays or powders.

### Deodorant preparation

According to the invention, combinations and preparations with further deodorant active ingredients and antiperspirant active ingredient such as aluminium salts are preferred. It is thus possible to achieve, inter alia, improved efficacy, improved water resistance, longer-lasting effect and improved acceptance by the consumer.

Preparations in the form of deodorants are, for example, aerosol sprays, roll-ons, deodorant sticks, powders, powder sprays, intimate cleansing compositions etc. The preparations according to the invention can also be combined with astringents such as preferably aluminium salts, e.g. aluminium oxychloride. It is thereby possible to control unpleasant body odour on a broader basis (reduction in the perspiration and prevention of the microbial degradation of the perspiration).

The preparations according to the invention can also be combined with fragrances which make a contribution to the deodorizing effect and increase the acceptance by the consumer.

### Preparation for the treatment of blemished skin, acne

A further embodiment of the invention is the use of the combination according to the invention for producing a (cosmetic or pharmaceutical) preparation for the treatment of blemished skin, acne. In this connection, the preparations typically comprise 0.05 to 5% by weight, preferably 0.1 to 2% by weight and particularly preferably 0.2 to 1% by weight, of the combination, in each case based on the total weight of the preparations, and also optionally one or more active ingredients, auxiliaries and/or additives.

Optional active ingredients, auxiliaries and/or additives of this type are preferably selected from active ingredients such as alpha-hydroxycarboxylic acids, benzoyl peroxide, linoleic acid, retinoids (tretinoin, isotretinoin), salicylic acid, zinc compounds etc., carrier substances such as water, lower alcohols (such as ethanol, isopropanol), polyols such as propylene glycol, glycerol, skin care fatty or fat-like substances such as oleic acid esters, fatty alcohols, thickeners such as hydroxyethylcellulose, hydroxypropylcellulose, polyacrylates, polyvinylpyrrolidone, cyclomethicones, emulsifiers (e.g. nonionogenic such as fatty alcohol polyethoxylates, ethoxylated sorbitan esters), long-chain polyglycol ethers, buffers (pH 4-9, preferably 5-7), ester oils (e.g. jojoba oil), silicone oils, oils (e.g. olive oil), perfume, dyes, antioxidants (e.g. vitamin E, BHT in amounts of from 0.01 to 0.05% by weight) or customary cosmetic or pharmaceutical base substances for producing O/W or W/O emulsions, gels, lotions or other preparation forms.

### Preparation for the treatment of dandruff

One preferred embodiment of the preparation according to the invention is an antidandruff hair cleansing composition, an antidandruff hair rinse or an antidandruff hair treatment which comprises the combination according to the invention in an amount of from 0.1 to 10% by weight, preferably 0.3 to 6% by weight, particularly preferably 0.5 to 2% by weight, based on the total weight of the preparation, and also optionally one or more active ingredients, auxiliaries and/or additives.

Optional active ingredients, auxiliaries and/or additives of this type are preferably selected from active ingredients such as ketoconazole, climbazole, clotrimazole, bifonazole, and undecylenic acid, octopirox, ciclopirox, zinc pyrithione, conjugated linoleic acid, selenium disulfide, salicylic acid etc.

Carriers include materials which are usually used in hair care products, such as water, solvents (e.g. ethanol, isopropanol), humectants, thickeners, perfume or fragrance, colourants, dyes, pigments, pearlizing agents, fatty alcohols, fatty acid esters, fatty amides, emulsifiers, silicones, viscosity modifiers, preservatives, complexing agents, such as ETDA, UV filters, amino acids and amino acid derivatives, proteins, protein hydrolyzates, carbohydrates, polysaccharides, plant extracts, buffers etc.

The present invention also provides a hair treatment composition which is to be rinsed out and has a content of the combination according to the invention upon whose application, after rinsing out with water, increased deposition of the contained active ingredients on hair and scalp takes place (setting, depot effect). In this connection, the combination according to the invention assists the care, shine-promoting and combability-improving effect of the composition. Also provided is a composition which remains on the skin, scalp or the hair after use.

Preferably, the hair care composition is a shampoo or a hair conditioning composition which typically comprises water in an amount of from about 50 to about 98% by weight, preferably 60 to 90% by weight, particularly preferably at least 70% by weight. The shampoo comprises one or more cleaning surfactants which are cosmetically compatible and are suitable for local application on the hair.

Examples of cleaning surfactants are anionic surfactants, such as alkyl sulphates, alkyl ether sulphates, alkylarylsulfonates, alkyl sulfosuccinates, N-alkyl sarcosinates, alkyl phosphates, alkyl ether phosphates, alkyl ether carboxylates, alpha-olefinsulfonates or salts thereof etc.; amphoteric surfactants such as alkylamine oxides, alkylbetaines, alkylamidopropylbetaines, alkylsulfobetaines, alkyl glycinates, alkyl amphopropionates etc.; nonionic surfactants (e.g. as cosurfactants), such as fatty alcohol ethoxylates, alkyl- or dialkylalkanolamides, alkyl polyglycosides (APGs) etc.; cationic surfactants, such as C8-C20-alkyltrimethylammonium salts, cetylpyridinium salts, benzalkonium salts, dialkyldimethylammonium salts etc.; cationic polymers such as, for example, polyquaternium grades, Luviquat grades, Gafquat grades, cationic polysaccharides, cationic cellulose derivatives etc.

### Preparation for the treatment of foot/skin fungus

A further embodiment of the invention is the use of the combination according to the invention for producing a (cosmetic or pharmaceutical) preparation for the treatment of foot/skin fungus. In this connection, the preparations typically comprise 0.05 to 10% by weight, preferably 0.1 to 5% by weight and particularly preferably 0.2 to 2% by weight, of the combination, in each case based on the total weight of the preparations, and also optionally one or more active ingredients, auxiliaries and/or additives.

Optional active ingredients, auxiliaries and/or additives of this type are preferably selected from active ingredients such as triclosan, farnesol (in particular to combat skin fungus in cosmetic preparations), etc.

The fungicidal active ingredients (antimycotics) used are, for example, compounds from the class of imidazoles, triazoles, benzylamines, allylamines, morpholines, phenols, quats etc., e.g. butenafin, terbinafin, ketoconazole, miconazole, bifonazole, clotrimazole, climbazole, econazole etc., also undecylenic acid and undecylenic acid derivatives.

Customary auxiliaries and additives used in preparations for controlling foot/skin fungus are solvents, such as water, lower alcohols, polyols, glycol ethers, polyethylene glycol, buffers, preservatives, humectants, complexing agents, antioxidants, emulsifiers, penetration promoters, thickeners, viscosity modifiers, perfume or fragrance, ointment bases, skin protectants, skin care agents, oils, plant extracts, polymers, essential oils, plant extracts, carbohydrates and carbohydrate derivatives, fatty alcohols, surface-active substances etc.

By using the combinations according to the invention it is surprisingly possible to effectively furnish preparations in a low-VOC or VOC-free manner. This contributes to the environmental friendliness and customer acceptance of the preparations. By contrast, example formulation D in DE 42 40 674 C1 comprises 40% by weight of ethanol.

It has been found, for example, that with a use concentration of 0.1% by weight of the above described particularly preferred concentrate (corresponds merely to 0.016% by weight of glycerol ether, such as 1-(2-ethylhexyl) glycerol ether, 0.0016% by weight of bispyridinium alkane (such as octenidine dihydrochloride), 0.0825% by weight of polyol (such as 1,2-propylene glycol) and about 80 ppm of antioxidant (such as vitamin E) an efficacy as deodorant active ingredient comparable with 0.1% by weight of triclosan is achieved. This was also surprising in view of the large amount of the active ingredients which are obligatorily prescribed in deodorant preparation D in DE 42 40 674 C1 (0.9% by weight of glycerol monoalkyl ether, 0.1% by weight of bispyridinium alkane and 1% by weight of 1,2-propylene glycol). It could not be deduced from DE 42 40 674 C1 that through the provision of combinations according to the invention, a deodorant active ingredient comparable with the undesired, chlorine-containing triclosan can be made available as concentrate.

The combinations according to the invention are storage-stable, i.e. the formation of undesired decomposition products and the degradation of active ingredient are not observed even upon storage at elevated temperature, such as at 40°C, over a period of 3 months, 6 months, 9 months or even 12 months.

The preparations according to the invention have comparatively selective efficacy and, at the low required use concentration, protect the microflora of the skin, which is important for the defence protection of opportunistic or pathogenic germs.

The preparations according to the invention are characterized by good skin compatibility, they are compatible with a large number of customary cosmetic auxiliaries and additives, they are adequately readily water-soluble (in contrast to many other deodorant active ingredients such as triclosan or antimycotically effective substances such as clotrimazole, ketoconazole, bifonazole, econazole, tioconazole, cyclopiroxolamine, terbinafin, amorolfin etc.) and do not accumulate on the skin. On the other hand, as a result of their amphiphilic character, they attach to constituents of skin and/or hair and are thus effective for longer.

The individual constituents of the preparations according to the invention are known as skin care additives and humectants (glycerol ethers such as Sensiva SC 50) and/or as wound disinfectants (bispyridinium alkanes such as octenidine) and, besides their deodorizing function, therefore assist the improvement in the skin condition or protect against infections (wound infection of relatively small skin injuries, in particular so-called superinfections of the skin by fungi and bacteria).

The preparations according to the invention protect against microorganisms (bacteria, yeasts, fungi) which penetrate into and/or inhabit the skin and hair folicles and symptoms such as flaking, itching or skin blemishes (eczema, greasy skin) or can adversely affect the wellbeing in another way.

The active ingredient combinations according to the invention can prevent the formation of seborrhoeic symptoms, in particular dandruff, and also eliminate existing seborrhoeic symptoms, in particular dandruff.

The preparations according to the invention protect against the cosmetic, dermatological and medical phenomena caused by these microorganisms (e.g. armpit odour, foot odour, body odour, dandruff, acne, superinfections, wound infections).

The preparations according to the invention reduce the adhesion of microorganisms to surface structures of living and dead cells of the human skin and in so doing counteract the undesired effects of the microorganisms on the skin.

The advantages of using the present active ingredient combinations over the prior art are the significantly increased efficacy compared with the required concentrations of individual active ingredient which achieve a comparable effect. This permits the development of milder, more skin friendly products.

The preparations according to the invention can be used as products with a broad pH range, the pH range is advantageously 3 to 9, preferably 4 to 8, particularly preferably 5 to 7.

## Claims

1. Active ingredient combination which consists in:
a) 8% to 30% by weight of 1-(2-ethylhexyl) glycerol ether or of 1-(dodecyl) glycerol ether,
b) 0.8% to 3% by weight of octenidine dihydrochloride,
c) 40% to 88% by weight of polyol is selected from 1,2-propylene glycol, glycerol, erythritol, 1,2,6-hexanetriol, inositol, lactitol, maltitol, mannitol, methylpropanediol, phytantriol, polyglycerols, sorbitol and xylitol, and
d) 0.003% to 0.02% by weight of antioxidant selected from vitamin E and its derivatives, 3-tert-butyl-4-hydroxyanisole and 2,6-di-tert-butyl-p-cresol, said active ingredient combination being free from aliphatic monohydric alcohol, from amine oxide, from aromatic alcohols and the weight ratio of the sum of components a) b) and d) to component c) being 1:2 to 1: 8.

2. Combination according to Claim 1, **characterized in that** the polyol is 1,2-propylene glycol.

3. Combination according to one of the preceding claims, **characterized in that** the antioxidant is selected from vitamin E and 3-tert-butyl-4-hydroxyanisole.

4. Combination according to one of the preceding claims, **characterized in that** it consists in:
a) 14 to 18% by weight of 1-(2-ethylhexyl) glycerol ether,
b) 1.4 to 1.8% by weight of octenidine dihydrochloride,
c) 70 to 85% by weight of 1,2-propylene glycol and
d) 0.006 to 0.010% by weight of vitamin E.

5. Combination according to one of the preceding claims for use in a method of treatment of undesired body odour.

6. Cosmetic preparation which comprises 0.05 to 1% by weight, of the combination according to one of claims 1 to 4, and optionally one or more further active ingredient(s), auxiliary(ies) and/or additive(s).

## Patentansprüche

1. Wirkstoffkombination, die aus Folgendem besteht:
a) 8 Gew.-% bis 30 Gew.-% 1-(2-Ethylhexyl)glycerinether oder 1-(Dodecyl)glycerinether,
b) 0,8 Gew.-% bis 3 Gew.-% Octenidin-Dihydrochlorid,
c) 40 Gew.-% bis 88 Gew.-% Polyol, das aus 1,2-Propylenglykol, Glycerin, Erythrit, 1,2,6-Hexantriol, Inosit, Lactit, Maltit, Mannit, Methylpropandiol, Phytantriol, Polyglycerinen, Sorbit und Xylit ausgewählt ist, und
d) 0,003 Gew.-% bis 0,02 Gew.-% Antioxidationsmittel, das aus Vitamin E und dessen Derivaten, 3-tert.-Butyl-4-hydroxyanisol und 2,6-Di-tert.-Butyl-p-kresol ausgewählt ist, wobei die Wirkstoffkombination frei von aliphatischem einwertigem Alkohol, von Aminoxid und von aromatischen Alkoholen ist und das Gewichtsverhältnis der Summe der Komponenten a), b) und d) zu der Komponente c) 1:2 bis 1:8 beträgt.

2. Kombination nach Anspruch 1, **dadurch gekennzeichnet, dass** das Polyol 1,2-Propylenglykol ist.

3. Kombination nach einem der vorhergehenden Ansprüche, **dadurch gekennzeichnet, dass** das Antioxidationsmittel aus Vitamin E und 3-tert.-Butyl-4-hydroxyanisol ausgewählt ist.

4. Kombination nach einem der vorhergehenden Ansprüche, **dadurch gekennzeichnet, dass** sie aus Folgendem besteht:
a) 14 bis 18 Gew.-% 1-(2-Ethylhexyl)glycerinether,
b) 1,4 bis 1,8 Gew.-% Octenidin-Dihydrochlorid,
c) 70 bis 85 Gew.-% 1,2-Propylenglykol und
d) 0,006 bis 0,010 Gew.-% Vitamin E.

5. Kombination nach einem der vorhergehenden Ansprüche zur Verwendung in einem Verfahren zur Behandlung von unerwünschtem Körpergeruch.

6. Kosmetisches Präparat, das 0,05 bis 1 Gew.-% der Kombination nach einem der Ansprüche 1 bis 4 und gegebenenfalls einen oder mehrere weitere Wirkstoff, Hilfsstoff und/oder Additiv umfasst.

## Revendications

1. Combinaison de principes actifs qui consiste en :
a) 8 % à 30 % en poids de 1-(2-éthylhexyl)glycéroléther ou de 1-(dodécyl)glycérol éther,
b) 0,8 % à 3 % en poids de dichlorhydrate d'octénidine,
c) 40 % à 88 % en poids d'un polyol choisi parmi le 1,2-propylène glycol, le glycérol, l'érythritol, le 1,2,6-hexanetriol, l'inositol, le lactitol, le maltitol, le mannitol, le méthylpropanediol, le phytantriol, les polyglycérols, le sorbitol et le xylitol, et
d) 0,003 % à 0,02 % en poids d'un antioxydant choisi parmi la vitamine E et ses dérivés, le 3-tert-butyl-4-hydroxyanisole et le 2,6-di-tert-butyl-p-crésol, ladite combinaison de principes actifs étant exempte d'alcool monohydrique aliphatique, d'oxyde d'amine, d'alcool aromatique et le rapport pondéral de la somme des composants a), b) et d) sur c) étant de 1/2 à 1/8.

2. Combinaison selon la revendication 1, **caractérisée en ce que** le polyol est le 1,2-propylène glycol.

3. Combinaison selon l'une quelconque des revendications précédentes, **caractérisée en ce que** l'antioxydant est choisi parmi la vitamine E et le 3-tert-butyl-4-hydroxyanisole.

4. Combinaison selon l'une quelconque des revendications précédentes, **caractérisée en ce qu'**elle consiste en :
a) 14 à 18 % en poids de 1-(2-éthylhexyl)glycéroléther,
b) 1,4 à 1,8 % en poids de dichlorhydrate d'octénidine,
c) 70 à 85 % en poids de 1,2-propylène glycol et
d) 0,006 à 0,010 % en poids de vitamine E.

5. Combinaison selon l'une quelconque des revendications précédentes pour son utilisation dans un procédé de traitement d'une odeur corporelle indésirable.

6. Préparation cosmétique qui comprend 0,05 à 1 % en poids, de la combinaison selon l'une quelconque des revendications 1 à 4, et facultativement un ou plusieurs autre(s) principe(s) actif(s), auxiliaire(s) et/ou additif(s).
